(19) Europäisches Patentamt
European Patent Office
Office européen des brevets

(11) Veröffentlichungsnummer: **0 065 724**
**B1**

# (12) EUROPÄISCHE PATENTSCHRIFT

(45) Veröffentlichungstag der Patentschrift: **08.08.84**

(21) Anmeldenummer: **82104261.1**

(22) Anmeldetag: **15.05.82**

(51) Int. Cl.³: **C 07 D 471/04**, C 07 D 487/04, A 01 N 25/32 // (C07D471/04, 235/00, 221/00),(C07D471/04, 239/00, 221/00),(C07D487/04, 235/00, 209/00),(C07D487/04, 239/00, 209/00)

## (54) Dihalogenacetamide, Verfahren zu ihrer Herstellung und herbizide Mittel, die Acetanilide als herbizide Wirkstoffe und diese Dihalogenacetamide als antagonistische Mittel enthalten.

(30) Priorität: **26.05.81 DE 3120859**

(43) Veröffentlichungstag der Anmeldung: **01.12.82 Patentblatt 82/48**

(45) Bekanntmachung des Hinweises auf die Patenterteilung: **08.08.84 Patentblatt 84/32**

(84) Benannte Vertragsstaaten: **AT BE CH DE FR GB IT LI NL**

(56) Entgegenhaltungen:
**EP - A - 0 031 042**
**DE - A - 1 802 468**
**DE - A - 2 218 097**
**DE - A - 2 245 471**
**DE - A - 2 620 101**

(73) Patentinhaber: **BASF Aktiengesellschaft, Carl-Bosch-Strasse 38, D-6700 Ludwigshafen (DE)**

(72) Erfinder: **Plath, Peter, Dr., Berner Weg 24, D-6700 Ludwigshafen (DE)**
Erfinder: **Hoffmann, Werner, Dr., Ringstrasse 11 C, D-6708 Neuhofen (DE)**
Erfinder: **Schwendemann, Volker, Dr., Hauptstrasse 64 A, D-6901 Wiesenbach (DE)**
Erfinder: **Wuerzer, Bruno, Dipl.-Landwirt Dr., Ruedigerstrasse 13, D-6701 Otterstadt (DE)**

**EP 0 065 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Dihalogenacetamide, Verfahren zu ihrer Herstellung und herbizide Mittel, die Acetanilide als herbizide Wirkstoffe und diese Dihalogenacetamide als antagonistische Mittel enthalten.

Acetanilide der Formel

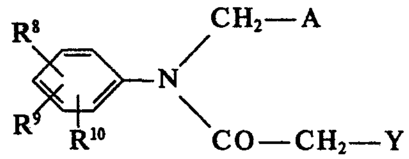

(II)

in der

- $R^8$ Wasserstoff, einen unverzweigten oder verzweigten Alkyl- oder Alkoxyrest mit bis zu 5 Kohlenstoffatomen,
- $R^9$ Wasserstoff, Halogen, einen unverzweigten oder verzweigten Alkyl- oder Alkoxyrest mit bis zu 5 Kohlenstoffatomen,
- $R^{10}$ Wasserstoff, Halogen, einen unverzweigten oder verzweigten Alkyl- oder Alkoxyrest mit bis zu 5 Kohlenstoffatomen,
- $R^8$ zusammen mit $R^9$ eine orthoständig verknüpfte, gegebenenfalls durch unverzweigte oder verzweigte Alkylgruppen mit bis zu 4 Kohlenstoffatomen substituierte Alkylenketten mit bis zu 6 Kohlenstoffatomen,
- Y Chlor oder Brom und
- A einen Alkoxy- oder Alkoxyalkylrest mit bis zu 4 Kohlenstoffatomen oder ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, wobei A auch für Salze der 2 oder 3 Stickstoffatome enthaltenden Azole stehen kann, bedeutet,

sind herbizid ausgezeichnet wirksam, führen aber bei Anwendung in Kulturen, wie Mais, oder auch in anderen Kulturen aus der Familie der Gramineen zu Schädigungen der Nutzpflanzen.

Aufgabe der Erfindung war es deshalb, antagonistische Mittel zu finden, die diese Unverträglichkeit der herbiziden Acetanilide gegenüber bestimmten Kulturpflanzen kompensieren.

Herbizide Mittel, die neben herbiziden Wirkstoffen Dichloracetamide als antagonistisch wirkende Verbindungen enthalten, sind aus der DE-OS 2 218 097 und der DE-OS 2 245 471 bekannt. Die in der DE-OS 2 218 097 beschriebenen Dichloracetamide werden vorwiegend zur Antagonisierung unerwünschter Kulturpflanzenschädigungen durch Thiolcarbamate verwendet, während aus der DE-OS 2 245 471 auch herbizide Mittel bekannt sind, die Dichloracetamide und Chloracetanilide, beispielsweise 2-Chlor-2',6'-diäthyl-N-butoxymethylacetanilid oder 2-Chlor-2 ',6'-diäthyl-N-methoxymethylacetanilid, enthalten.

Es wurde gefunden, daß Dihalogenacetamide der Formel

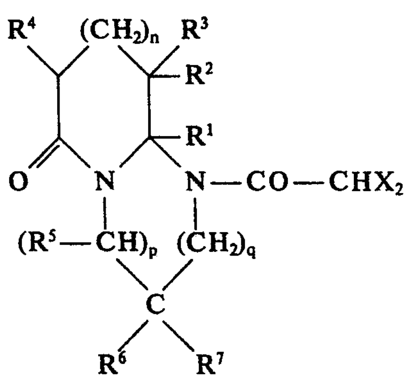

(I)

in der $R^1$, $R^2$, $R^3$, $R^4$, $R^5$, $R^6$ und $R^7$ gleich oder verschieden sind und für Wasserstoff oder Methyl stehen und X Chlor oder Brom, n 0 oder 1, p 0, 1 oder 2 und q 0, 1 oder 2 bedeuten, mit der Maßgabe, daß $R^2$, $R^3$ und $R^4$ nicht gleichzeitig Wasserstoff bedeuten, sich ausgezeichnet zur Steigerung der Kulturpflanzenverträglichkeit von herbiziden Acetaniliden der Formel II eignen. Herbizide Mittel, die ein Acetanilid der Formel II und ein Dihalogenacetamid der Formel I enthalten, können sowohl in Mais als auch in anderen Getreidekulturen eingesetzt werden. Dabei bleibt die gute herbizide Wirkung der Acetanilide erhalten, während Schädigungen an den Nutzpflanzen ganz oder weitgehend unterbunden werden.

Bevorzugt sind antagonistische Mittel der Formel I, bei denen $R^1$ und $R^4$ für Wasserstoff stehen,

wenn $R^2$ und $R^3$ Methyl bedeuten, $R^4$ für Methyl steht, wenn $R^2$ und $R^3$ Wasserstoff bedeuten, und $R^5$, $R^6$ und $R^7$ Wasserstoff, n, p und q 1 und X Chlor bedeuten.

Beispiele für antagonistisch wirkende Dihalogenacetamide der Formel I sind Dichloracetamide oder Dibromacetamide von

7-Methyl-8-oxo-1,4-diazabicyclo[3.3.0]octan,
5,7-Dimethyl-8-oxo-1,4-diazabicyclo[3.3.0]octan,
2,7-Dimethyl-8-oxo-1,4-diazabicyclo[3.3.0]octan,
3,7-Dimethyl-8-oxo-1,4-diazabicyclo[3.3.0]octan,
2,3,7—Trimethyl-8-oxo-1,4-diazabicyclo[3.3.0]octan,
8-Methyl-9-oxo-1,5-diazabicyclo[4.3.0]nonan,
6,8-Dimethyl-9-oxo-1,5-diazabicyclo[4.3.0]nonan,
3,3,8-Trimethyl-9-oxo-1,5-diazabicyclo[4.3.0]nonan,
3,3,6,8-Tetramethyl-9-oxo-1,5-diazabicyclo[4.3.0]nonan,
3-Methyl-2-oxo-1,7-diazabicyclo[4.3.0]nonan,
3,5-Dimethyl-2-oxo-1,7-diazabicyclo[4.3.0]nonan,
3,6-Dimethyl-2-oxo-1,7-diazabicyclo[4.3.0]nonan,
5,5-Dimethyl-2-oxo-1,7-diazabicyclo[4.3.0]nonan,
3, 6,8-Trimethyl-2-oxo-1,7-diazabicyclo[4.3.0]nonan,
3,6,9-Trimethyl-2-oxo-1,7-diazabicyclo[4.3.0]nonan,
3,6,8,9-Tetramethyl-2-oxo-1,4-diazabicyclo[4.3.0]nonan,
9-Methyl-10-oxo-1,5-diazabicyclo[4.4.0]decan,
6,9-Dimethyl-10-oxo-1,5-diazabicyclo[4.4.0]decan,
7,9-Dimethyl-10-oxo-1,5-diazabicyclo[4.4.0]decan,
3,3,9-Trimethyl-10-oxo-1,5-diazabicyclo[4.4.0]decan,
3,3,6,9-Tetramethyl-10-oxo-1,5-diazabicyclo[4.4.0]decan,
7,7-Dimethyl-10-oxo-1,5-diazabicyclo[4.4.0]decan,
3,3,7,7-Tetramethyl-10-oxo-1,5-diazabicyclo[4.4.0]decan,
3-Methyl-2-oxo-1,6-diazabicyclo[5.3.0]decan,

Bevorzugte antagonistische Mittel sind insbesondere

7-Dichloracetyl-3,6-dimethyl-2-oxo-1,7-diazabicyclo[4.3.0]nonan,
7-Dichloracetyl-5,5-dimethyl-2-oxo-1,7-diazabicyclo[4.3.0]nonan,
5-Dichloracetyl-8-methyl-9-oxo-1,5-diazabicyclo[4.3.0]nonan,
4-Dichloracetyl-7-methyl-8-oxo-1,4-diazabicyclo[4.3.0]octan.

Dihalogenacetamide der Formel I können durch Umsetzung von Aminen der Formel

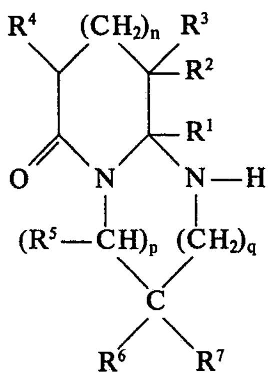

(III)

in der $R^1$ bis $R^7$, n, p und q die obengenannten Bedeutungen haben, mit Dihalogenacetylchloriden der Formel $X_2CH-COCl$, in der X Chlor oder Brom bedeutet, in Gegenwart eines halogenwasserstoffbindenden Mittels und eines Lösungs- oder Verdünnungsmittels erhalten werden. Die Umsetzung wird bei einer Temperatur im Bereich zwischen $-10$ und $+20°C$ durchgeführt.

Als Verdünnungs- oder Lösungsmittel kommen gegebenenfalls halogenierte Kohlenwasserstoffe, wie Toluol, Xylole, Chlorbenzol, Dichlormethan, Ethylenchlorid, Ether, wie Diethylether, Methyltert.-butylether, Tetrahydrofuran, 1,4-Dioxan, oder Nitrile, wie Acetonitril, in Betracht.

Geeignete halogenwasserstoffbindende Mittel sind Alkalimetallcarbonate, Alkalimetallhydrogencarbonate, wäßrige Lösungen von Alkalimetallhydroxiden, Trialkylamine, N,N-Dialkylaniline, wie N,N-Dimethylanilin, und Pyridinbasen. Das Amin der Formel III wird zweckmäßigerweise mit der äquimolaren Menge oder der 1,2fachen molaren Menge an Dihalogenacetylchlorid umgesetzt. Pro Mol Dihalogenacetylchlorid werden 1 bis 1,2 Mol halogenwasserstoffbindendes Mittel zugegeben.

Für den Fall, daß X Chlor bedeutet, können die erfindungsgemäßen Dihalogenacetamide auch durch Umsetzung von Diazabicycloalkanen der Formel III mit Chloralhydrat in Gegenwart eines säurebindenden Mittels und katalytischer Mengen Cyanid, das z. B. in Form von Natriumcyanid oder Acetoncyanhydrin zugegeben wird, erhalten werden (DE-OS 2 807 340).

Die bicyclischen Amine der Formel II sind zum Teil aus der DE-OS 1 802 468 bekannt. Sie lassen sich nach dem dort beschriebenen Herstellungsverfahren durch Umsetzung von $\gamma$-Oxo- bzw. $\delta$-Oxo-carbonsäuren oder deren Estern mit $\alpha,\omega$-Alkylendiaminen erhalten.

Das zur Herstellung von 7-Methyl-8-oxo-1,4-diazabicyclo-[3.3.0]octan benötigte Vorprodukt 2-Methyl-3-formyl-propionsäuremethylester ist in bekannter Weise durch Hydroformylierung von Methacrylsäuremethylester zugänglich. Der zur Synthese von 3,6-Dimethyl-2-oxo-1,7-diazabicyclo[4.3.0]nonan benötigte 2-Methyl-5-oxo-hexansäuremethylester ist durch Addition von Aceton an Methacrylsäuremethylester erhältlich. Durch Addition von Isobutylaldehyd an Acrylsäuremthylester wird der 4,4-Dimethyl-5-oxo-pentansäure-methylester erhalten.

## Herstellung der Amine der Formel III

Man mischt 63,2 g (0,4 Mol) 2-Methyl-5-oxo-hexansäuremethylester mit 200 ml Ethanol und gibt anschließend unter Rühren und Kühlen 72 g (1,2 Mol) Ethylendiamin zu. Danach erhitzt man 8 Stunden lang zum Sieden, läßt abkühlen und destilliert das Reaktionsgemisch. Man erhält 47 g (70% d. Th.) eines Öles vom Kp. = 115° C/0,9 mbar, das nach NMR-Analyse folgende Struktur hat:

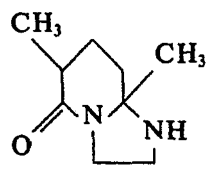

($C_9H_{16}N_2O$)

In gleicher Weise erhält man folgende Verbindungen der Formel III:

| | $R^1$ | $R^2$ | $R^3$ | $R^4$ | $R^5$ | $R^6$ | $R^7$ | n | p | q | Kp. |
|---|---|---|---|---|---|---|---|---|---|---|---|
| | H | H | H | $CH_3$ | H | H | H | 0 | 1 | 0 | 99° C/0,26 mbar |
| | H | H | H | $CH_3$ | H | H | H | 0 | 1 | 1 | 103° C/0,4 mbar |
| | H | H | H | $CH_3$ | H | $CH_3$ | $CH_3$ | 0 | 1 | 1 | 112° C/0,8 mbar |
| | $CH_3$ | H | H | $CH_3$ | H | H | H | 1 | 1 | 1 | 120° C/0,8 mbar |
| | $CH_3$ | H | H | $CH_3$ | H | $CH_3$ | $CH_3$ | 1 | 1 | 1 | 122° C/1,3 mbar |
| | H | $CH_3$ | $CH_3$ | H | H | H | H | 1 | 1 | 1 | 127° C/0,13 mbar |
| | H | $CH_3$ | $CH_3$ | H | H | H | H | 1 | 0 | 1 | 125° C/0,13 mbar |
| | H | $CH_3$ | $CH_3$ | H | H | $CH_3$ | $CH_3$ | 1 | 1 | 1 | 128° C/0,13 mbar |
| Gemisch aus | H | H | H | $CH_3$ | $CH_3$ | H | H | 0 | 1 | 0 | 101° C/0,26 mbar |
| und | H | H | H | $CH_3$ | H | $CH_3$ | H | 0 | 1 | 0 | |
| Gemisch aus | $CH_3$ | H | H | $CH_3$ | $CH_3$ | H | H | 1 | 1 | 0 | 117° C/0,7 mbar |
| und | $CH_3$ | H | H | $CH_3$ | H | $CH_3$ | H | 1 | 1 | 0 | |
| Gemisch aus | H | $CH_3$ | $CH_3$ | H | $CH_3$ | H | H | 1 | 1 | 0 | 122° C/0,13 mbar |
| und | H | $CH_3$ | $CH_3$ | H | H | H | $CH_3$ | 1 | 1 | 0 | |

Herstellung der Dihalogenacetamide der Formel I

Zu einer Lösung von 0,1 Mol des Diazabicycloalkanons der Formel III in 150 ml Toluol gibt man 5 g (0,125 Mol) NaOH in 10 ml Wasser. Anschließend tropft man unter Eiskühlung 0,11 Mol Dichloracetylchlorid oder Dibromacetylchlorid zu und läßt 12 bis 16 Stunden lang nachrühren. Zur Aufarbeitung versetzt man mit 100 ml Wasser, rührt kurz durch und trennt die Toluolphase ab. Die Toluollösung wird nach Waschen mit Natriumhydrogencarbonatlösung und danach mit Wasser abgetrennt, über Natriumsulfat getrocknet und anschließend unter vermindertem Druck eingeengt. Nach Anreiben mit Ether wird das Dihalogenacetamid in kristalliner Form erhalten.

Im manchen Fällen erhält man das Produkt als Öl. Dann wird das Produkt zweckmäßigerweise durch Chromatographie an Kieselgel gereinigt; Laufmittel ist z. B. Essigsäureethylester.

Folgende Dihalogenacetamide der Formel I werden beispielsweise nach der oben beschriebenen Umsetzung erhalten:

| Nr. | $R^1$ | $R^2$ | $R^3$ | $R^4$ | $R^5$ | $R^6$ | $R^7$ | X | n | p | q | Fp. [°C] |
|---|---|---|---|---|---|---|---|---|---|---|---|---|
| 1 | H | H | H | $CH_3$ | H | H | H | Cl | 0 | 1 | 0 | 88—89 |
| 2 | H | H | H | $CH_3$ | H | H | H | Cl | 0 | 1 | 1 | Öl |
| 3 | H | H | H | $CH_3$ | H | $CH_3$ | $CH_3$ | Cl | 0 | 1 | 1 | 109—111 |
| 4 | $CH_3$ | H | H | $CH_3$ | H | H | H | Cl | 1 | 1 | 0 | 153—154 |
| 5 Gemisch aus | $CH_3$ | H | H | $CH_3$ | $CH_3$ | H | H | Cl | 1 | 1 | 0 | 180—182 |
| und | $CH_3$ | H | H | $CH_3$ | H | $CH_3$ | H | Cl | 1 | 1 | 0 | |
| 6 | $CH_3$ | H | H | $CH_3$ | H | H | H | Cl | 1 | 1 | 1 | 140—141 |
| 7 | $CH_3$ | H | H | $CH_3$ | H | $CH_3$ | $CH_3$ | Cl | 1 | 1 | 1 | 123—124 |
| 8 | H | $CH_3$ | $CH_3$ | H | H | H | H | Cl | 1 | 0 | 1 | 128—130 |
| 9 Gemisch aus | H | $CH_3$ | $CH_3$ | H | $CH_3$ | H | H | Cl | 1 | 1 | 0 | 124—126 |
| und | H | $CH_3$ | $CH_3$ | H | H | H | $CH_3$ | Cl | 1 | 1 | 0 | |
| 10 | H | $CH_3$ | $CH_3$ | H | H | $CH_3$ | $CH_3$ | Cl | 1 | 1 | 1 | |
| 11 | H | H | H | $CH_3$ | H | H | H | Cl | 0 | 1 | 2 | |
| 12 | H | H | H | $CH_3$ | H | H | H | Br | 0 | 1 | 1 | |
| 13 | $CH_3$ | H | H | $CH_3$ | H | H | H | Br | 1 | 0 | 1 | |
| 14 | H | $CH_3$ | $CH_3$ | H | H | H | H | Br | 1 | 1 | 0 | |
| 15 | H | H | H | $CH_3$ | $CH_3$ | $CH_3$ | H | Cl | 0 | 1 | 0 | |
| 16 | $CH_3$ | H | H | $CH_3$ | $CH_3$ | $CH_3$ | H | Cl | 1 | 1 | 0 | |

Acetanilide, deren Kulturpflanzenverträglichkeit durch die neuen Dichloracetamide der Formel I verbessert werden kann, sind solche der Formel II, bei denen $R^8$ für Wasserstoff, Alkyl bis zu 5 Kohlenstoffatomen wie Methyl, Ethyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Ethoxy, Propoxy, Butoxy, Pentyloxy;
$R^9$ und $R^{10}$ für Wasserstoff, Halogen, wie Fluor, Chlor, Brom oder Jod, Alkyl bis zu 5 Kohlenstoffatomen, wie Methyl, Ethyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Ethoxy, Propoxy, Butoxy, Pentyloxy;
$R^8$ zusammen mit $R^9$ für eine orthoständig verknüpfte, gegebenenfalls durch Alkyl mit bis zu 4 Kohlenstoffatomen substituierte Alkylenkette mit bis zu 6 Kohlenstoffatomen, wie Ethylen, Trimethylen, Tetramethylen, 1-Methyl-trimethylen, 1,1-Dimethyl-trimethylen, 1,1-Dimethyl-tetramethylen;
Y für Chlor oder Brom, vorzugsweise Chlor, und
A für ein über ein Ring-Stickstoffatom gebundenes Azol, wie Pyrrol, Pyrazol, Imidazol, 1,2,4-Triazol, 1,2,3-Triazol, Tetrazol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, oder für einen Alkoxy- oder Alkoxyalkylrest mit bis zu 4 Kohlenstoffatomen, wie Methoxy, Ethoxy, Methoxymethyl, 2-Methoxyethyl, stehen.

Beispiele für substituierte Azole für A sind

2,6-Dimethylpyrrol, Tetramethylpyrrol, 3(5)-Methylpyrazol, 4-Methylpyrazol, 3(5)-Ethylpyrazol, 4-Ethylpyrazol, 3(5)-Isopropylpyrazol, 4-Isopropylpyrazol, 3,5-Dimethylpyrazol, 3,4,5-Trimethylpyrazol, 3(5)-Phenylpyrazol, 4-Phenylpyrazol, 3,5-Diphenylpyrazol, 3(5)-Phenyl-5(3)-methylpyrazol, 3(5)-Chlorpyrazol, 4-Chlorpyrazol, 4-Brompyrazol, 3,5.-Dimethyl-4-chlorpyrazol, 3,5-Dimethyl-4-brompyrazol, 4-Chlor-3(5)-methylpyrazol, 4-Methyl-3,5-dichlorpyrazol, 3(5)-Methyl-4,5(3)-dichlorpyrazol, 3(5)-Chlor-5(3)-methylpyrazol, 4-Methoxypyrazol, 3(5)-Methyl-5(3)-trifluormethylpyrazol, 3(5)-Methyl-5(3)-ethoxycarbonylpyrazol, 3(5)-Methyl-5(3)-methylthio-4-methoxy-carbonylpyrazol, 4-Cyanopyrazol, 4,5-Dichlor-imidazol, 2-Methyl-4,5-dichlorimidazol, 3(5)-Methyl-1,2,4-triazol, 3,5-Dimethyl-1,2,4-triazol, 3(5)-Chlor-1,2,4-triazol, 3,5-Dichlor-1,2,4-triazol, 4(5)-Methyl-1,2,3-triazol, 5-Methyltetrazol oder 5-Chlortetrazol.

Darüber hinaus kann der Rest A für den Fall, daß das Azol 2 oder 3 Stickstoffatome enthält, auch salzartig an eine der üblichen starken anorganischen oder organischen Säuren, wie Chlorwasserstoffsäure, Salpetersäure, Schwefelsäure, Trichloressigsäure, Methansulfonsäure, Perfluorhexansulfonsäure oder Dodecylbenzolsulfonsäure gebunden sein.

Bevorzugt sind Acetanilide der Formel II, die in 2- und 6-Stellung am Phenylring Methyl oder Ethyl und in 3-Stellung Wasserstoff oder Methyl tragen. Y steht vorzugsweise für Chlor, während für A insbesondere Ethoxy oder Methoxymethyl oder Azolylreste, wie Pyrazolyl, 3,5-Dimethylpyrazolyl, 1,2,4-Triazolyl oder 4,5-Dichlorimidazolyl in Betracht kommen.

Insbesondere enthalten die erfindungsgemäßen herbiziden Mittel folgende Acetanilide:

2-Chlor-2′,6-dimethyl-N-(pyrazol-1-yl-methyl)-acetanilid,
2-Chlor-2′-methyl-6′-ethyl-N-(pyrazol-1-yl-methyl)-acetanilid,
2-Chlor-2′,6′-dimethyl-N-(3,5-dimethylpyrazol-1-yl-methyl)-acetanilid,
2-Chlor-2′,6′-dimethyl-N-(1,2,4-triazol-1-yl-methyl)-acetanilid,
2-Chlor-2′,3′,6′-trimethyl-N-(pyrazol-1-yl-methyl)-acetanilid,
2-Chlor-2′-methyl-6′-ethyl-N-(3,5-dimethyl-pyrazol-1-yl-methyl)-acetanilid,
2-Chlor-2′,6′-diethyl-N-(3,5-dimethyl-pyrazol-1-yl-methyl)-acetanilid,
2-Chlor-2′,3′,6′-trimethyl-N-(3,5-dimethyl-pyrazol-1-yl-methyl)-acetanilid,
2-Chlor-2′,6′-diethyl-N-(pyrazol-1-yl-methyl)-acetanilid,
2-Chlor-2′,6′-dimethyl-N-(4,5-dichlorimidazol-1-yl-methyl)-acetanilid,
2-Chlor-2′-methyl-6′-ethyl-N-(4,5-dichlorimidazol-1-yl-methyl)-acetanilid,
2-Chlor-2′,6′-diethyl-N-(4,5-dichlorimidazol-1-yl-methyl)-acetanilid,
2-Chlor-2′-methyl-6′-ethyl-N-(1,2,4-triazol-1-yl-methyl)-acetanilid,2-Chlor-2′,6′-diethyl-N-(1,2,4-triazol-1-yl-methyl)-acetanilid,
2-Chlor-2′,3′,6′-trimethyl-N-(1,2,4-triazol-1-yl-methyl)-acetanilid,2-Chlor-2′,6′-dimethyl-N-(2-methoxy-ethyl)-acetanilid, 2-Chlor-2′-methyl-6′-ethyl-N-ethoxymethyl-acetanilid,
2-Chlor-2′,6′-diethyl-N-(n-butoxymethyl)-acetanilid,
2-Chlor-2′-ethyl-6′-methyl-N-(1-methyl-2-methoxy-ethyl)-acetanilid und
2-Chlor-2′,6′-dimethyl-N-[(2-methyl-pyrazol-5-yl)-methyl]-acetanilid.

Die Acetanilide der Formel II und ihre Herstellung sind Gegenstand der DE-OS 2 648 008, der DE-OS 2 744 396, der DE-OS 2 305 495, der DE-OS 2 328 340, der DE-OS 2 842 315 und der US 3 547 620.

Herbizide Wirkstoffe und antagonistisch wirkende Verbindungen können gemeinsam oder getrennt vor oder nach der Saat in den Boden eingearbeitet werden. Bei den Acetaniliden ist das gebräuchlichste Anwendungsverfahren die Ausbringung auf die Erdoberfläche unmittelbar nach Ablage der Samen oder in der Zeit zwischen Einsaat und Auflaufen der jungen Pflanzen. Auch eine Behandlung während des Auflaufens ist möglich. In jedem Fall kann das antagonistisch wirkende Mittel gleichzeitig mit dem herbiziden Wirkstoff ausgebracht werden. Auch eine getrennte Ausbringung, wobei der Antagonist

zuerst und anschließend der herbizide Wirkstoff oder umgekehrt auf das Feld gebracht werden, ist möglich, sofern zwischen Ausbringung beider Substanzen nicht soviel Zeit verstreicht, daß der herbizide Wirkstoff bereits Schaden an den Kulturpflanzen anrichtet. Wirkstoff und Antagonist können hierbei als Spritzmittel in suspendierbarer, emulgierbarer oder löslicher Form oder als Granulate getrennt oder gemeinsam formuliert vorliegen. Denkbar ist auch eine Behandlung der Kulturpflanzensamen mit dem Antagonisten vor der Aussaat. Der herbizide Wirkstoff wird dann allein in der üblichen Weise appliziert.

Für das gleiche herbizide Acetanilid werden unterschiedliche Mengen einer antagonistisch wirkenden Verbindung benötigt, wenn das Acetanilid in verschiedenen Kulturen eingesetzt wird. Die Mengenverhältnisse, in denen Acetanilid und Dihalogenacetamid eingesetzt werden, sind in breiten Bereichen variabel. Sie sind abhängig von der Struktur des Acetanilids, des Dihalogenacetamids und der jeweiligen Kultur.

Die neuen herbiziden Mittel können neben Acetanilid und Dihalogenacetamid weitere herbizide oder wachstumsregulierende Wirkstoffe anderer chemischer Struktur enthalten, wobei der antagonistische Effekt erhalten bleibt. Sie können beispielsweise 2-Chlor-4-ethylamino-6-isopropylamino-1,3,5-triazin, 2-(2-Chlor-4-ethylamino-1,3,5-triazin-6-yl-amino)-2-methyl-propionitril, N-(1-Ethyl-n-propyl)-2,6-dinitro-3,4-dimethylanilin oder 1-(4-Isopropyl-phenyl)-3,3-dimethylharnstoff enthalten.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthhalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenyl, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver , Streu- und Stäubmittel können durch Mischen oder gemeinsames Vermahlen von herbizidem Wirkstoff und/oder Antidot mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent an herbizidem Wirkstoff und Antidot, vorzugsweise zwischen 0,5 und 90 Gewichtsprozent. Die Aufwandmengen an herbizidem Wirkstoff betragen 0,2 bis 5 kg/ha. Diese Menge an herbizidem Wirkstoff wird, gemeinsam oder getrennt, mit einer solchen Menge an Antidot ausgebracht, daß das Anteilsverhältnis herbizider Wirkstoff : antagonistischer Verbindung 1 : 2 bis 1 : 0,01, vorzugsweise 1 : 0,25 bis 1 : 0,05, Gewichtsteile beträgt.

Beispiele für Formulierungen sind:

I. 40 Gewichtsteile der Mischung aus vier Gewichtsteilen 2-Chlor-2',6'-dimethyl-N-(pyrazol-1-yl-methyl)-acetanilid und einem Gewichtsteil 7-Dichloracetyl-3,6-dimethyl-2-oxol-1,7-diazabicyclo[4.3.0]nonan werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

II. 3 Gewichtsteile der Mischung aus einem Gewichtsteil 2-Chlor-2'-methyl-6'-ethyl-N-(pyrazol-1-yl-methyl)-acetanilid und einem Gewichtsteil 7-Dichloracetyl-5,5-dimethyl-2-oxo-1,7-diazabicyclo[4.3.0]nonan werden mit 97 Gewichtsteilen feinteiligem Kaolin innig vermischt. Man erhält auf diese Weise ein Stäubemittel, das 3 Gewichtsprozent des Wirkstoffs enthält.

III. 30 Gewichtsteile der Mischung aus einem Gewichtsteil 2-Chlor-2'-methyl-6'-ethyl-N-(1,2,4-triazol-1-yl-methyl)-acetanilid und zwei Gewichtsteilen 5-Dichloracetyl-8-methyl-9-oxo-1,5-diazabicyclo[4.3.0]nonan werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Teile der Mischung aus 8 Gewichtsteilen 2-Chlor-2'-methyl-6'-ethyl-N-ethoxymethyl-acetanilid und einem Gewichtsteil 4-Dichloracetyl-7-methyl-8-oxo-1,4-diazabicyclo[4.3.0]nonan werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

V. 20 Gewichtsteile der Mischung aus 10 Gewichtsteilen 2-Chlor-2',6'-dimethyl-N-(2-methoxyethyl)-acetanilid und einem Gewichtsteil 4-Dichloracetyl-7-methyl-8-oxo-1,4-diazabicyclo[4.3.0]nonan werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Die Wirkung der erfindungsgemäßen herbiziden Mittel auf das Wachstum von erwünschten und unerwünschten Pflanzen im Vergleich zu dem herbiziden Mittel aus den gleichen herbiziden Wirkstoffen und einer antagonistisch wirkenden, bereits bekannten Verbindung chemisch ähnlicher Struktur wird durch die folgenden biologischen Beispiele belegt. Die Versuche zeigen, daß die Verträglichkeit der herbiziden Acetanilide durch kombinierte Anwendung mit den neuen Dihalogenacetamiden bei gleichbleibender herbizider Wirkung entscheidend verbessert wird.

Die Versuchsserien wurden im Gewächshaus durchgeführt.

Plastikpikierkisten von 51 cm Länge, 32 cm Breite und 6 cm Höhe wurden mit lehmigem Sand von pH 6 bis 7 und etwa 1,5 bis 3% Humus gefüllt. In dieses Substrat wurde Mais (Zea mays) in Reihen flach eingesät. Außerdem wurde Echinochloa crus-galli als unerwünschte Pflanze breitwürfig hinzugegeben. Der nicht sterilisierte Boden enthielt zusätzlich lebensfähige Unkrautsamen, welche zur Population unerwünschter Pflanzen beitrugen. Dadurch wurde ein mit Kulturpflanzen bestellter und mit Unkräutern verseuchter Acker simuliert.

Wirkstoffe und Antagonisten wurden sowohl einzeln als auch in den beschriebenen Mischungen aufgebracht. Dazu verteilte man sie, emulgiert oder suspendiert in Wasser als Trägermedium, und spritzte sie mittels fein verteilender Düsen unmittelbar nach der Saat und vor Auflaufen der Testpflanzen auf die Erdoberfläche. In einigen Fällen wurden die Mittel auch vor Einsaat der Kulturpflanzen in den Boden eingemischt. Bei der Samenbehandlung puderte man die Samen mit einer festen Formulierung der Wirkstoffe oder man tränkte die Samen der Kulturpflanzen über einen gewissen Zeitraum in einer flüssigen Aufbereitung (Suspension, Emulsion, Lösungen) der antagonistischen Verbindungen (Samenquellung, seed soaking), trocknete sie so weit, daß die Samen nicht aneinanderklebten und säte sie danach aus. Auch eine Inkrustierung der Samen ist möglich (seed coating). Nach Einsaat der Samen und Behandlung mit dem Herbizid wurden die Kisten beregnet und mit durchsichtigen Plastikhauben abgedeckt, bis die Pflanzen aufgelaufen waren. Durch diese Maßnahmen wurde ein gleichmäßiges Keimen und Anwachsen der Pflanzen ermöglicht. Die Kisten werden in einem Temperaturbereich von durchschnittlich 15 bis 25° C aufgestellt.

Die so angelegten Gewächshausversuche wurden beobachtet, bis der Mais 3 bis 5 Blätter entwickelt hatte. Nach diesem Stadium waren Schädigungen durch die herbiziden Mittel nicht mehr zu erwarten.

Die Wirkung der Mittel wurde nach einer Skala von 0 bis 100 vorgenommen. Hierbei bedeutet 0 bei normaler Aufgang und Entwicklung der Pflanzen, bezogen auf die unbehandelte Kontrolle. 100 entsprach einem völligen Ausbleiben der Keimung bzw. Absterben der Pflanzen.

Die antagonistische Wirkung der Dihalogenacetamide wurde am Beispiel des 2-Chlor-2',6'-dimethyl-N-(pyrazol-1-yl-methyl)-acetanilid (A) gezeigt. Als Vergleichsmittel dienten herbizide Mittel, die neben dem herbiziden Acetanilid das aus der DE-OS 2 218 097 bekannte N-Dichloracetyl-morpholin (V) als antagonistisches Mittel enthielten.

Tabelle 1

Verbesserung der Verträglichkeit für Mais durch antagonistische Verbindungen bei Vorauflaufanwendung im Gewächshaus

| Herbizider Wirkstoff | Antagonistische Verbindung Nr. | Aufwandmenge [kg/ha] | Schädigung [%] bei Zea mays |
|---|---|---|---|
| A | – | 2,0 | 80 |
| A | 6 | 2,0+0,5 | 10 |
| A | 3 | 12,0+0,5 | 20 |
| A | 8 | 2,0+0,5 | 20 |

0 = normaler Aufgang, keine Schädigung
100 = Pflanzen nicht aufgelaufen oder abgestorben

Tabelle 2

Verbesserung der Verträglichkeit für Mais durch antagonistische Verbindungen bei Vorauflaufanwendung im Gewächshaus

| Herbizider Wirkstoff | Antagonistische Verbindung Nr. | Aufwandmenge [kg/ha] | Testpflanzen und Schädigung [%] Zea mays | Echinochloa crus-galli |
|---|---|---|---|---|
| A | – | 1,0 | 64 | 100 |
| A | 4 | 1,0+0,25 | 4 | 100 |
| | | 1,0+0,125 | 4 | 100 |
| A | V | 1,0+0,25 | 19 | 100 |

0 = normaler Aufgang, keine Schädigung
100 = Pflanzen nicht aufgelaufen oder abgestorben

Tabelle 3

Verbesserung der Maisverträglichkeit im Gewächshausversuch durch Saatgutbehandlung

| Herbizider Wirkstoff | Antagonistische Verbindung Nr. | Dosierung | Methode | Testpflanzen und Schädigung [%] Zea mays | Echinochloa crus-galli |
|---|---|---|---|---|---|
| A | – | 1,0 | VAS*) | 70 | 100 |
| A | 4 | 1,0 | VAS*) | 0 | 100 |
| | | +50 g pro 100 kg Saatgut**) | als Samenbeizung | | |

*) VAS = Vorauflaufspritzung nach der Einsaat der Kultur
**) des Maises

## Patentansprüche

1. Dihalogenacetamide der Formel

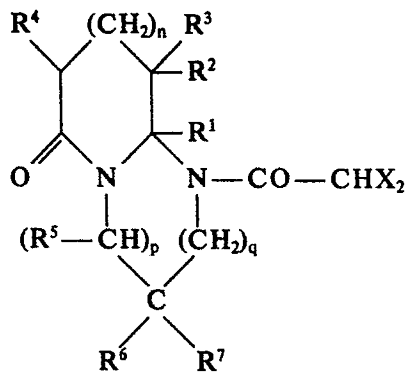

(I)

in der $R^1$, $R^2$, $R^3$, $R^4$, $R^5$, $R^6$ und $R^7$ gleich oder verschieden sind und für Wasserstoff oder Methyl stehen und X Chlor oder Brom, n 0 oder 1, p 0, 1 oder 2 und q 0, 1 oder 2 bedeuten, mit der Maßgabe, daß $R^2$, $R^3$ und $R^4$ nicht gleichzeitig Wasserstoff bedeuten.

2. Dihalogenacetamide der Formel I gemäß Anspruch 1, dadurch gekennzeichnet, daß $R^1$ Wasserstoff, $R^2$ und $R^3$ Methyl, $R^4$, $R^5$, $R^6$ und $R^7$ Wasserstoff, X Chlor und n, p und q 1 bedeuten.

3. Dihalogenacetamide der Formel I gemäß Anspruch 1, dadurch gekennzeichnet, daß $R^1$, $R^2$ und $R^3$ Wasserstoff, $R^4$ Methyl, $R^5$, $R^6$ und $R^7$ Wasserstoff, X Chlor und n, p und q 1 bedeuten.

4. Verfahren zur Herstellung von Dichloracetamiden der Formel

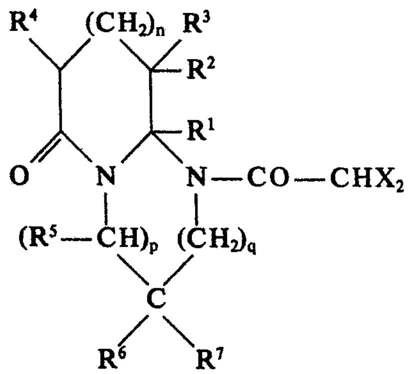

(I)

in der $R^1$, $R^2$, $R^3$, $R^4$, $R^5$, $R^6$ und $R^7$ gleich oder verschieden sind und für Wasserstoff oder Methyl stehen und X Chlor oder Brom, n 0 oder 1, p 0, 1 oder 2 und q 0, 1 oder 2 bedeuten, mit der Maßgabe, daß $R^2$, $R^3$ und $R^4$ nicht gleichzeitig Wasserstoff bedeuten, dadurch gekennzeichnet, daß man Diazabicycloalkane der Formel

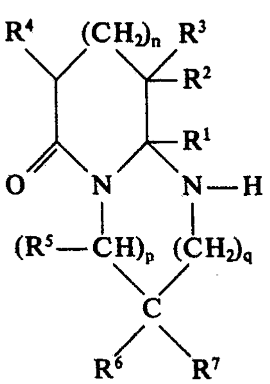

(III)

in der $R^1$, $R^2$, $R^3$, $R^4$, $R^5$, $R^6$, $R^7$, n, p und q die oben angegebenen Bedeutungen haben, mit einem Dihalogenacetylchlorid der Formel $XCH_2COCl$, in der X Chlor oder Brom bedeutet, in Gegenwart eines chlorwasserstoffbindenden Mittels in einem Lösungs- oder Verdünnungsmittel umsetzt.

5. Herbizide Mittel, gekennzeichnet durch einen Gehalt an mindestens einem Acetanilid der Formel

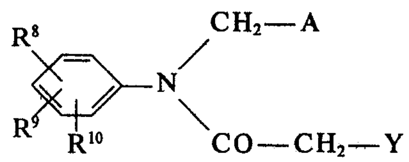

(II)

in der

$R^8$ Wasserstoff, einen unverzweigten oder verzweigten Alkyl- oder Alkoxyrest mit bis zu 5 Kohlenstoffatomen,

$R^9$ Wasserstoff, Halogen, einen unverzweigten oder verzweigten Alkyl- oder Alkoxyrest mit bis zu 5 Kohlenstoffatomen,

$R^{10}$ Wasserstoff, Halogen, einen unverzweigten oder verzweigten Alkyl- oder Alkoxyrest mit bis zu 5 Kohlenstoffatomen,

$R^8$ zusammen mit $R^9$ eine orthoständig verknüpfte, gegebenenfalls durch unverzweigte oder verzweigte Alkylgruppen mit bis zu 4 Kohlenstoffatomen substituierte Alkylenkette mit bis zu 6 Kohlenstoffatomen,

Y Chlor oder Brom und

A einen Alkoxy- oder Alkoxyalkylrest mit bis zu 4 Kohlenstoffatomen oder ein über ein Ringstickstoffatom gebundenes Azol, das einfach oder mehrfach durch Halogen, Phenyl, Alkyl-, Alkoxy-, Alkylthio- oder Perfluoralkylrest mit jeweils bis zu 4 Kohlenstoffatomen, Cyan, Carboxy oder Alkoxycarbonyl mit bis zu 4 Kohlenstoffatomen in der Alkoxygruppe substituiert sein kann, wobei A auch für Salze der 2 oder 3 Stickstoffatome enthaltenden Azole stehen kann, bedeutet,

als herbizidem Wirkstoff und mindestens einem Dihalogenacetamid der Formel

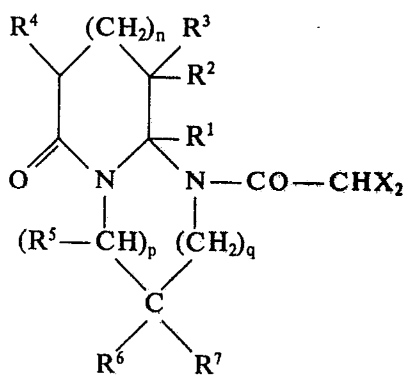

(I)

in der $R^1$, $R^2$, $R^3$, $R^4$, $R^5$, $R^6$ und $R^7$ gleich oder verschieden sind und für Wasserstoff oder Methyl stehen und X Chlor oder Brom, n 0 oder 1, p 0, 1 oder 2 und q 0, 1 oder 2 bedeuten, mit der Maßgabe, daß $R^2$, $R^3$ und $R^4$ nicht gleicheitig Wasserstoff bedeuten, als antagonistischem Mittel.

6. Herbizides Mittel nach Anspruch 5, dadurch gekennzeichnet, daß es als herbiziden Wirkstoff 2-Chlor-2',6'-dimethyl-N-(pyrazol-l-yl-methyl)-acetanilid enthält.

7. Herbizides Mittel nach Anspruch 5, dadurch gekennzeichnet, daß das Anteilsverhältnis Acetanilid : Dihalogenacetamid bei gemeinsamer oder getrennter Ausbringung 1 : 2 bis 1 : 0,01 Gewichtsteile beträgt.

8. Verfahren zur selektiven Bekämpfung von unerwünschtem Pflanzenwuchs, dadurch gekennzeichnet, daß man herbizide Mittel nach Anspruch 5 vor, bei oder nach Aussaat der Kulturpflanzen, vor oder während des Auflaufens der Kulturpflanzen ausbringt.

9. Verfahren zur selektiven Bekämpfung von unerwünschtem Pflanzenwuchs, dadurch gekennzeichnet, daß man Acetanilide der Formel II gemäß Anspruch 5 und Dihalogenacetamide der Formel I gemäß Anspruch 1 vor, bei oder nach der Aussaat der Kulturpflanzen, vor oder während des Auflaufens der Kulturpflanzen gleichzeitig oder nacheinander in beliebiger Reihenfolge ausbringt.

10. Verfahren zur Verhinderung von Schädigungen von Kulturpflanzen durch herbizide Acetanilide der Formel II gemäß Anspruch 5, dadurch gekennzeichnet, daß man das Saatgut der Kulturpflanzen mit einer antagonistisch wirkenden Menge eines Dihalogenacetamids der Formel I gemäß Anspruch 1 behandelt.

**Claims**

1. A dihaloacetamide of the formula

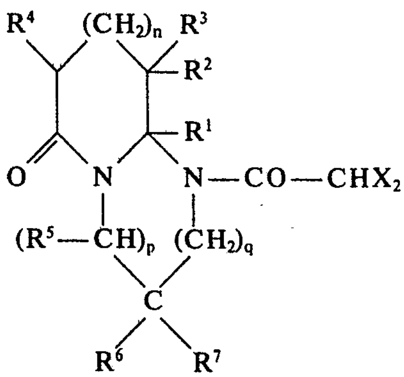

(I)

where $R^1$, $R^2$, $R^3$, $R^4$, $R^5$, $R^6$ and $R^7$ are identical or different and each is hydrogen or methyl, X is chlorine or bromine, n is 0 or 1, p is 0, 1 or 2 and q is 0, 1 or 2, with the proviso that $R^2$, $R^3$ and $R^4$ do not simultaneously denote hydrogen.

2. A dihaloacetamide of the formula I as claimed in claim 1, where $R^1$ is hydrogen, $R^2$ and $R^3$ are methyl, $R^4$, $R^5$, $R^6$ and $R^7$ are hydrogen, X is chlorine, and n, p and q are 1.

3. A dihaloacetamide of the formula I as claimed in claim 1, where $R^1$, $R^2$, and $R^3$ are hydrogen, $R^4$ is methyl, $R^5$, $R^6$ and $R^7$ are hydrogen, X is chlorine, and n, p and q are 1.

4. A process for the preparation of a dihaloacetamide of the formula

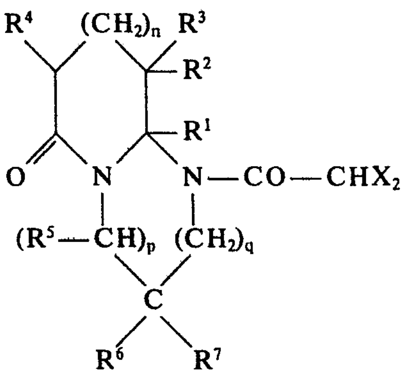

(I)

where $R^1$, $R^2$, $R^3$, $R^4$, $R^5$, $R^6$ and $R^7$ are identical or different and each is hydrogen or methyl, X is chlorine or bromine, n is 0 or 1, p is 0, 1 or 2 and q is 0, 1 or 2 with the proviso that $R^2$, $R^3$ and $R^4$ do not simultaneously denote hydrogen, wherein a diazabicycloalkane of the formula

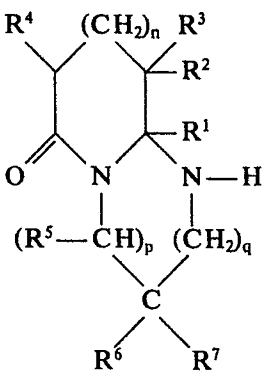

(III)

where $R^1$, $R^2$, $R^3$, $R^4$, $R^5$, $R^6$, $R^7$, n, p and q have the above meanings, is reacted with a dihaloacetyl chloride of the formula $X_2CH-COCl$, where X is chlorine or bromine, in the presence of a hydrogen halide acceptor in a solvent or diluent.

5. A herbicidal agent containing, as herbicidal active ingredient, at least one acetanilide of the formula

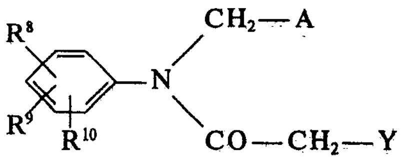

(II)

where $R^8$ is hydrogen or straight-chain or branched alkyl or alkoxy of up to 5 carbon atoms, $R^9$ is hydrogen, halogen or straight-chain or brannched alkyl or alkoxy of up to 5 carbon atoms, and $R^{10}$ is hydrogen, halogen or straight-chain or branched alkyl or alkoxy of up to 5 carbon atoms, or $R^8$ together with $R^9$ is alkylene of up to 6 carbon atoms which is linked in the ortho-position and is unsubstituted or substituted by straight-chain or branched alkyl of up to 4 carbon atoms, Y is chlorine or bromine, and A is alkoxy or alkoxyalkyl of up to 4 carbon atoms or azolyl which is bonded via a ring nitrogen atom and may be monosubstituted or polysubstituted by halogen or phenyl, or by alkyl, alkoxy, alkylthio or perfluoroalkyl, each having up to 4 carbon atoms, or by cyano or carboxyl, or by alkoxycarbonyl, where alkoxy has up to 4 carbon atoms, and A can also be a salt of an azolyl radical containing 2 or 3 nitrogen atoms, and, as antagonist, a dihaloacetamide of the formula

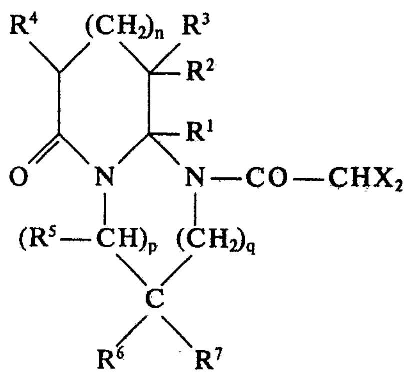

(I)

where $R^1$, $R^2$, $R^3$, $R^4$, $R^5$, $R^6$ and $R^7$ are identical or different and each is hydrogen or methyl, X is chlorine or bromine, n is 0 or 1, p is 0, 1 or 2 and q is 0, 1 or 2, with the proviso that $R^2$, $R^3$ and $R^4$ do not simultaneously denote hydrogen.

6. A herbicidal agent as claimed in claim 5, where the herbicidal active ingredient is 2-chloro-2′,6′-dimethyl-N-(pyrazol-1-yl-methyl)-acetanilide.

7. A herbicidal agent as claimed in claim 5, where the weight ratio of acetanilide to dihaloacetamide, applied either separately or together, is from 1 : 2 to 1 : 0.01.

8. A process for the selective control of unwanted plant growth, wherein a herbicidal agent as claimed in claim 6 is applied before, during or after sowing of the crop plants, or before or during emergence of the crop plants.

9. A process for the selective control of unwanted plant growth, wherein an acetanilide of the formula II as claimed in claim 5 and a dihaloacetamide of the formula I as claimed in claim 1 are applied — simultaneously or one after the other in any order — before, during or after sowing of the crop plants, or before or during emergence of the crop plants.

10. A process for the prevention of demage to crop plants by herbicidal acetanilides of the formula II as claimed in claim 5, wherein the seed of the crop plants is treated with an antagonistically effective amount of a dihaloacetamide of the formula I as claimed in claim 1.

**Revendications**

1. Dihalo-acétamides de la formule

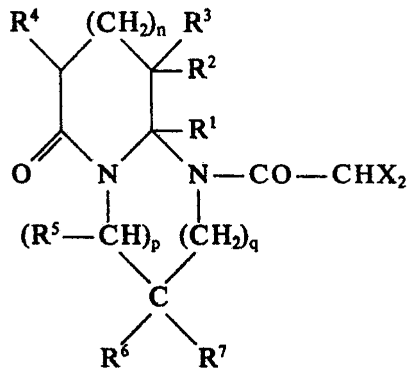

(I)

dans laquelle $R^1$, $R^2$, $R^3$, $R^4$, $R^5$, $R^6$ et $R^7$ peuvent être identiques ou différents et représentent chacun un atome d'hydrogène ou un radical méthyle, X désigne un atome de chlore ou de brome, n = 0 ou 1, p = 0, 1 ou 2 et q = 0, 1 ou 2, $R^2$, $R^3$, et $R^4$ ne pouvant cependant désigner simultanément de l'hydrogène.

2. Dihalo-acétamides de la formule I selon la revendication 1, caractérisés en ce que $R^1 = H$, $R^2 = R^3 =$ méthyle, $R^4 = R^5 = R^6 = R^7 = H$, $X = Cl$ et $n = p = q = 1$.

3. Dihalo-acétamides de la formule I selon la revendication 1, caractérisés en ce que $R^1 = R^2 = R^3 = H$, $R^4 =$ méthyle, $R^5 = R^6 = R^7 = H$, $X = Cl$ et $n = p = q = 1$.

4. Procédé de préparation de dichlor-acétamides de la formule

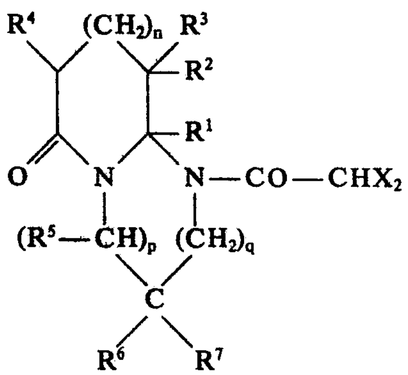

(I)

dans laquelle $R^1$, $R^2$, $R^3$, $R^4$, $R^5$, $R^6$ et $R^7$ peuvent être identiques ou différents et représentent chacun un atome d'hydrogène ou un radical méthyle, X désigne un atome de chlore ou de brome, n = 0 ou 1, p = 0, 1 ou 2 et q = 0, 1 ou 2, $R^2$, $R^3$ et $R^4$ ne pouvant cependant désigner simultanément de l'hydrogène, caractérisé en ce que l'on fait réagir des diaza-bicycloalcanes de la formule

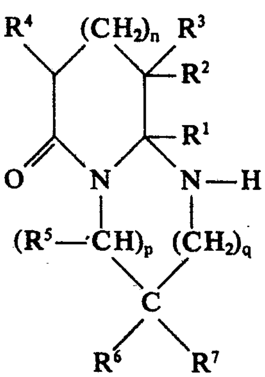

(III)

dans laquelle $R^1$, $R^2$, $R^3$, $R^4$, $R^5$, $R^6$, $R^7$ et n, p et q possèdent les significations définies ci-dessus, dans un solvant ou diluant et en présence d'un agent fixant l'hydrogène chloré, avec un chlorure de dihalo-acétyle de la formule $XCH_2COCl$, dans laquelle X représente du chlore ou du brome.

5. Composition herbicide, contenant comme substance herbicide au moins un acétanilide de la formule

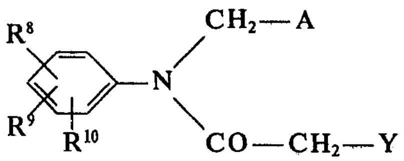

(II)

dans laquelle

$R^8$ désigne un atome d'hydrogène ou un radical alkyle ou alcoxy ramifié ou non avec jusqu'à cinq atomes de carbone,

$R^9$ désigne un atome d'hydrogène ou d'halogène ou un radical alkyle ou alcoxy ramifié ou non avec jusqu'à cinq atomes de carbone,

$R^{10}$ représente un atome d'hydrogène ou d'halogène ou un radical alkyle ou alcoxy ramifié ou non avec jusqu'à cinq atomes de carbone,

$R^8$ et $R^9$ constituent une chaîne alkylène avec jusqu'à six atomes de carbone, éventuellement substituée par des radicaux alkyle ramifiés ou non avec jusqu'à quatre atomes de carbone, fixée en position ortho,

Y représente un atome de chlore ou de brome et

A un groupe alcoxy ou alcoxy-alkyle avec jusqu'à quatre atomes de carbone ou un azole fixé par un atome d'azote du noyau cyclique et éventuellement mono- ou poly-substitué par des halogènes, des groupes phényle ou alkyle, alcoxy, alkyl-thio ou perfluor-alkyle avec jusqu'à quatre atomes de carbone, cyano, carboxy ou alcoxy-carbonyle avec un radical alcoxy avec jusqu'à quatre atomes de carbone,

A pouvant en outre représenter un sel d'un azole comprenant deux ou trois atomes d'azote,

et comme agent antagoniste au moins un dihalo-acétamide de la formule

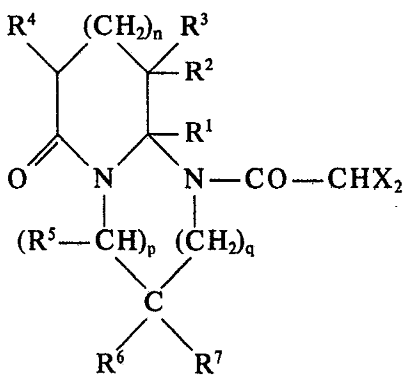

(I)

dans laquelle $R^1$, $R^2$, $R^3$, $R^4$, $R^5$, $R^6$ et $R^7$ peuvent être identiques ou différents et représentent chacun un atome d'hydrogène ou un radical méthyle, X désigne un atome de chlore ou de brome, $n = 0$ ou 1, $p = 0$, 1 ou 2 et $q = 0$, 1 ou 2, $R^2$, $R^3$ et $R^4$ ne pouvant cependant désigner simultanément de l'hydrogène.

6. Composition herbicide suivant la revendication 5, caractérisée en ce qu'elle contient en tant que substance herbicide du 2-chloro-2',6'-diméthyl-N-(pyrazol-1-méthyl)-acét-anilide.

7. Composition herbicide suivant la revendication 5, caractérisée en ce que les proportions (parties en poids) de l'acét-anilide et du dihalo-acét-amide, appliqués ensemble ou séparément, se situent dans un rapport compris entre 1 : 2 et 1 : 0,01.

8. Procede de lutte sélective contre le développement de plantes indédsirables, caractérisé en ce quel'on applique avant, pendant ou après l'ense,emcement ou avant ou pendant l'émergence de plantes de culture une compositon herbicide suivant la revendication 5.

9. Procédé de lutte sélective contre le développement de plantes indésirables, caractérisé en ce que l'on applique, avant, pendant ou après l'ensemencement ou avant ou pendant l'émergence de plantes de culture des acéanilides de la formula II suivant la revendication 5 et des dihalo-acét-amides de la formule I suivant la revendication 1, soit simultanénement, soit successivement dans un ordre quelconque.

10. Procédé pour éviter lers dommages aux plantes de culture, provoqués par des acét-anilides herbicides de la formule II suivant la revendication 5, caractérisé en ce que l'on traite les semences des plantes de culture une quantité ayant un effet antagoniste d'un dihalo-acétamide de la formule I suivant la revendication 1.